# EUROPÄISCHE PATENTANMELDUNG

(11) **EP 3 415 610 A1**
(43) Veröffentlichungstag der Anmeldung: **19.12.2018**
(21) Anmeldenummer: 18176289.9
(22) Anmeldetag: 06.06.2018
(51) Int. Cl.: C12M 1/107, C01B 3/00, C12M 1/00, H01M 8/0656, H01M 16/00, H01M 8/04082

(54) **VERFAHREN ZUR UMRÜSTUNG EINER BIOGASANLAGE**

(30) Priorität: 13.06.2017 DE 102017113028
(71) Anmelder: N-ERGIE Aktiengesellschaft, 90429 Nümberg (DE)
(72) Erfinder: Fleischer, Dr. Patrick, 91207 Lauf an der Pegnitz (DE)
(74) Vertreter: Ellwanger, Arndt

(57) **Zusammenfassung**

Die vorliegende Erfindung betrifft ein Verfahren zur Umrüstung einer Biogasanlage in eine Anlage zum Lagern von Wasserstoff, umfassend zwei oder gegebenenfalls drei Verfahrensschritte. Im ersten Verfahrensschritt wird das Innere von mindestens einem Fermenter (F1) der Biogasanlage gereinigt. Im optionalen zweiten Verfahrensschritt wird gegebenenfalls mindestens eine Schutzauskleidung im Inneren des gereinigten Fermenters (F1) angebracht. Im dritten Verfahrensschritt wird ein mit Wasserstoff beladener Träger (T2) in den gereinigten Fermenter (F1), der, sofern der zweite Verfahrensschritt durchgeführt worden ist, in seinem Inneren mit einer Schutzauskleidung versehen ist, eingebracht und gelagert. Weiterhin betrifft die vorliegende Erfindung einen Fermenter (F1) als solchen, der im Inneren mit mindestens einer Schutzauskleidung versehen ist.

## Beschreibung

Die vorliegende Erfindung betrifft ein Verfahren zur Umrüstung einer Biogasanlage in eine Anlage zum Lagern von Wasserstoff, umfassend zwei oder gegebenenfalls drei Verfahrensschritte. Im ersten Verfahrensschritt wird das Innere von mindestens einem Fermenter (F1) der Biogasanlage gereinigt. Im optionalen zweiten Verfahrensschritt wird gegebenenfalls mindestens eine Schutzauskleidung im Inneren des gereinigten Fermenters (F1) angebracht. Im dritten Verfahrensschritt wird ein mit Wasserstoff beladener Träger (T2) in den gereinigten Fermenter (F1), der, sofern der zweite Verfahrensschritt durchgeführt worden ist, in seinem Inneren mit einer Schutzauskleidung versehen ist, eingebracht und gelagert. Weiterhin betrifft die vorliegende Erfindung einen Fermenter (F1) als solchen, der im Inneren mit mindestens einer Schutzauskleidung versehen ist.

Biogasanlagen als solche sind bereits seit langem bekannt (siehe beispielsweise die freie Enzyklopädie Wikipedia unter dem Begriff "Biogasanlage"; https://de.wikipedia.org/wiki/biogasanlage; Version vom 19. April 2017). Eine Biogasanlage dient zur Erzeugung von Biogas durch Vergärung von Biomasse. Biogasanlagen werden in der Regel in landwirtschaftlichen Betrieben eingesetzt, wo beispielsweise tierische Exkremente (Gülle/Festmist) oder Energiepflanzen als Substrat eingesetzt werden. Als Nebenprodukt wird häufig ein als Gärrest bezeichneter Dünger produziert. Das bei der Biogasanlage als Hauptprodukt anfallende Biogas wird häufig zur dezentral gekoppelten Strom- und Wärmeerzeugung (Kraft-Wärme-Kopplung) in Blockheizkraftwerken genutzt. Dabei wird das Biogas, das gegebenenfalls noch getrocknet und/oder entschwefelt wird, einem Gasmotor zugeführt, der das Biogas in elektrischen Strom sowie gegebenenfalls in Wärme verwandelt. Der so produzierte Strom wird in das Netz eingespeist. Meistens erfolgt die Herstellung des elektrischen Stroms aus dem Biogas direkt vor Ort als Bestandteil einer Biogasanlage. Alternativ ist es auch möglich, dass das in einer Biogasanlage erzeugte Biogas auf Erdgasqualität gereinigt und als Biomethan (Bioerdgas) in das Erdgasnetz eingespeist wird.

Zentraler Bestandteil einer solchen Biogasanlage ist in der Regel ein Biogasreaktor, der auch als Fermenter bezeichnet wird (siehe auch Wikipedia; https://de.wikipedia.org/wiki/bioreaktor; Version vom 21. April 2017). In diesem Fermenter wird in Abhängigkeit von der eingebrachten Biomasse durch spezifische biologische Prozesse (Biokonversion, Biokatalyse) das Biogas erzeugt. In der Praxis gibt es zahlreiche Hersteller von solchen Reaktoren/Fermentern, die sich hinsichtlich Größe und Ausstattung sowie Art und Dimension der Zuleitungen der im Fermenter enthaltenen Einbauten, wie Rührwerke oder etwaige vorhandene Heizsysteme im Inneren des Fermenters, unterscheiden. Eine Biogasanlage kann auch zwei oder mehrere Fermenter enthalten. Weitere Fermenter werden auch als Nachgärer bezeichnet/eingesetzt. Weiterhin verfügen Biogasanlagen häufig über zahlreiche Vorrats- oder Lagerbehälter, um beispielsweise die zu vergärende Biomasse oder den Gärrückstand zu lagern. Die einzelnen Vorrichtungselemente einer Bioanlage sind in der Regel durch ein entsprechendes Leitungssystem sowie durch Pumpen miteinander verbunden.

DE-A 10 2014 010 641 betrifft einen Fermenter zur Erzeugung von Biogas durch anaerobe Fermentation mit mindestens einer Fermentierkammer und mit mindestens einer Zuführeinrichtung, wobei die Zuführeinrichtung zum Eintragen von Fermentiergut in die Fermentierkammer ausgebildet ist. Die Fermenter können darüber hinaus ein Rührwerkzeug aufweisen. Weiterhin wird eine Anlage zur Erzeugung von Biogas mit mindestens einem Fermenter sowie ein Verfahren zur Erzeugung von Biogas offenbart. Die Anlage zur Erzeugung von Biogas kann in ein Blockheizkraftwerk mit einem Verbrennungsmotor integriert werden.

Ein wesentliches Problem von Biogasanlagen ist deren wirtschaftliche Betreibung. Zumindest in Deutschland, aber auch in anderen europäischen Ländern sinngemäß, wird der Betrieb von Biogasanlagen im Rahmen der erneuerbaren Energien wirtschaftlich gefördert. Allerdings sind diese staatlichen Förderungen im Zusammenhang mit der Erzeugung von erneuerbaren Energien nicht unbegrenzt erhältlich, sondern werden in absehbarer Zeit, insbesondere im Zusammenhang mit dem Betrieb von Biogasanlagen, reduziert oder sogar auslaufen. Ohne staatliche Hilfe ist es für viele landwirtschaftliche Betriebe schwierig, insbesondere kleinere Biogasanlagen weiterhin wirtschaftlich zu betreiben. Ein weiteres Problem beim Betreiben von Biogasanlagen ist in der Gefahr von Gasleakagen bzw. dem Wärmeverlust zu sehen. Um das in der Regel brennbare Biogas zu handhaben bzw. zu lagern sind erhöhte Sicherheitsvorkehrungen notwendig.

DE-A 10 2012 005 023 offenbart eine Anordnung sowie ein Verfahren zur autonomen Bereitstellung von Elektrizität über Wasserstoff. Hierbei wird zunächst elektrischer Strom aus einer regenerativen Quelle bereitgestellt, der zur Herstellung von Wasserstoff aus Wasser in mindestens einem Elektrolyseur verwendet wird. Der so hergestellte Wasserstoff wird in einen ersten chemischen Reaktor überführt, der mindestens ein Substrat mit einem ausgedehnten pi-konjugierten System enthält, wobei in diesem ersten chemischen Reaktor eine zumindest teilweise Hydrierung des Substrats durchgeführt wird. Anschließend wird dieses zumindest teilweise hydrierte Substrat in einen Speichertank überführt. Aus diesem Speichertank wird das zumindest teilweise hydrierte Substrat wiederum in einen zweiten chemischen Reaktor überführt, wo eine Dehydrierung dieses Substrates unter Freisetzung von Wasserstoff erfolgt. Dieser im zweiten chemischen Reaktor erzeugte Wasserstoff wird in eine Anlage oder Maschine zur Wandlung des Wasserstoffs in elektrische Energie, vorzugsweise in eine Brennstoffzelle, überführt. Von dort wird elektrischer Strom an den Ort des Bedarfs abgegeben.

Das in DE-A 10 2012 005 023 beschriebene Verfahren bzw. die entsprechende Anlage eignet sich beispielsweise als Notstromaggregat oder an Orten, an denen ein öffentliches Stromnetz fehlt, sowie in Krisensituationen, wie Kriegen. Anwendungsgebiete können Krankenhäuser, chemische Anlagen, Computerzentralen oder auch Kernkraftwerke sein. Letztendlich wird bei diesem Verfahren Wasserstoff zwischengespeichert, was gegebenenfalls auch über einen längeren Zeitraum erfolgen kann, um ihn im Bedarfsfall zur Stromerzeugung einzusetzen. Der anfänglich bereitgestellte elektrische Strom, der aus regenerativen Quellen stammt, mit dem der Wasserstoff erzeugt wird, kann beispielsweise aus Wasserkraft, Windenergie, solarer Strahlung oder auch aus Biogas stammen. Die Durchführung des Verfahrens im Rahmen einer Biogasanlage ist in DE-A 10 2012 005 023 jedoch nicht offenbart.

Ein sinngemäßes Verfahren bzw. eine entsprechende Anordnung ist in DE-A 10 2011 121 704 offenbart. Die Anordnung (Vorrichtung) wird insbesondere zur Pufferung von Überschuss-Elektrizität in Gebäuden eingesetzt. Das entsprechende Verfahren dient zur Energiespeicherung in Gebäuden. Das Verfahren sowie die entsprechende Anordnung erfordern zwingend die Verwendung einer Brennstoffzelle zur Oxidation des in dem zweiten chemischen Reaktor freigesetzten Wasserstoffs unter Freisetzung von Energie.

DE-A 10 2014 223 427 offenbart ein Verfahren sowie die entsprechende Anlage zum Erzeugen und Speichern von Wasserstoff. In dem Verfahren wird zunächst ein Rohstoffgasgemisch bereitgestellt, das mindestens eine Kohlenwasserstoffverbindung aufweist. Dieses Rohstoffgasgemisch wird vorbehandelt, um ein Zwischengasgemisch mit erhöhtem Wasserstoffanteil herzustellen. Anschließend erfolgt ein Kontaktieren des Zwischengasgemisches mit einen Wasserstoff-Trägermedium zum Hydrieren des Wasserstoff-Trägermediums.

DE-B 10 2013 223 589 betrifft eine Anlage sowie ein entsprechendes Verfahren zum Speichern von Energie. Die Anlage umfasst eine Stromerzeugungseinheit, eine Wasserstofferzeugungseinheit, eine Wasserstoffspeicherungsvorrichtung, eine Wärmeerzeugungseinheit sowie eine Wärmespeicherungseinheit. Die Wasserstoffspeicherungsvorrichtung umfasst wiederum eine Beladeeinheit zum Beladen eines Trägermediums mit dem in der Wasserstofferzeugungseinheit erzeugten Wasserstoff sowie eine Entladeeinheit zum Entladen des Wasserstoffs von dem beladenen Trägermedium. Die Wärmespeicherungseinheit ist mit der Entladeeinheit zum Bereitstellen von Wärme verbunden. Weiterhin ist die Stromerzeugungseinheit mit der Wärmeerzeugungseinheit zur Bereitstellung von elektrischem Strom für die Wärmeerzeugung verbunden.

Die der vorliegenden Erfindung zugrunde liegende Aufgabe besteht in der Bereitstellung eines neuen Verfahrens zur Umrüstung einer Biogasanlage, um auf diese Weise Energie, insbesondere Wasserstoff, zu lagern. Die Energie, insbesondere der Wasserstoff, soll zu einem späteren Zeitpunkt wiedergewonnen werden können, insbesondere in Form von elektrischem Strom und gegebenenfalls von Wärme.

Gelöst wird die Aufgabe durch ein Verfahren zur Umrüstung einer Biogasanlage in eine Anlage zum Lagern von Wasserstoff umfassend die nachfolgenden Schritte a), c) und gegebenenfalls b):
a) Reinigen des Inneren von mindestens einem Fermenter (F1) der Biogasanlage,
b) gegebenenfalls Anbringen von mindestens einer Schutzauskleidung im Inneren des gereinigten Fermenters (F1),
c) Einbringen und Lagern eines mit Wasserstoff beladenen Trägers (T2) in den gegebenenfalls im Inneren mit einer Schutzauskleidung versehenen gereinigten Fermenter (F1).

Ein wesentlicher Vorteil des erfindungsgemäßen Verfahrens ist darin begründet, dass die jeweilige Infrastruktur der in Deutschland bzw. Europa zahlreich vorhandenen Biogasanlagen verwendet werden kann, um erfindungsgemäß Energie in Form von Wasserstoff zu speichern bzw. zu lagern. Dadurch können die Anfangsinvestitionskosten gegenüber einem kompletten Neubau/dem Kauf einer kompletten neuen Anlage deutlich reduziert werden. Wesentliche Vorrichtungskomponenten jeder gängigen Biogasanlage können für das erfindungsgemäße Verfahren verwendet werden, ohne dass dabei Kosten im großen Umfang im Zusammenhang mit der Umrüstung einzelner Vorrichtungselemente einer Biogasanlage anfallen müssen. Die anfallenden Kosten sind im Wesentlichen auf die Umrüstung (des Inneren) von mindestens einem Fermenter von Biogasbetrieb auf eine Vorrichtung bzw. Anlage zum Lagern von Wasserstoff beschränkt. Weiterhin können Vorrichtungselemente, die entweder direkt zur Biogasanlage gehören oder ihr unmittelbar angeschlossen sind, wie ein Blockheizkraftwerk oder ein darin enthaltener Gasmotor, auch ohne weitere Umrüstung direkt im erfindungsgemäßen Verfahren sinngemäß zum Betrieb bei einer Biogasanlage mit nachfolgender Stromerzeugung verwendet werden.

Ebenfalls ist es als vorteilhaft anzusehen, dass das erfindungsgemäße Verfahren auch parallel und/oder zeitversetzt zum herkömmlichen Betrieb einer Biogasanlage durchgeführt werden kann. So ist es denkbar, dass zusätzlich zum bisherigen "konventionellen Betrieb" einer Biogasanlage mindestens ein Fermenter in seinem Inneren umgerüstet wird, um das erfindungsgemäße Verfahren parallel oder zeitversetzt zur eigentlichen Produktion von Biogas bzw. der nachfolgenden Strom- oder Wärmeerzeugung durchgeführt wird. Bei dieser Verfahrensvariante ist es weiterhin vorteilhaft, wenn der aus elektrischem Strom erzeugte Wasserstoff zumindest teilweise zur Erzeugung von Biogas, insbesondere im Rahmen einer Methanisierung, eingesetzt wird.

Alternativ ist es selbstverständlich auch möglich, dass eine bisher konventionell genutzte Biogasanlage vollumfänglich zur Durchführung des erfindungsgemäßen Verfahrens umgerüstet wird. Dies ist insbesondere im Fall von kleineren Biogasanlagen, die im landwirtschaftlichen Bereich betrieben werden, von Interesse, weil deren wirtschaftliche Betreibung aufgrund des zumindest in Deutschland unmittelbar bevorstehenden Endes der wirtschaftlichen Förderung solcher Biogasanlagen sehr schwierig bis unmöglich ist.

Ein weiterer Vorteil gegenüber dem konventionellen Betrieb von Biogasanlagen ist im erfindungsgemäßen Verfahren darin zu sehen, dass es sehr sicher betrieben werden kann und eine saisonale Speicherung von Energie möglich ist. Bei konventionellen Biogasanlagen wird das vordergründig erzeugte Biogas in der Regel gleich zur Energieerzeugung in Form von elektrischem Strom und/oder Wärme weiterverarbeitet. Eine Speicherung von Biogas ist schwierig und kostenintensiv, da das in der Regel brennbare Biogas unter erhöhten Sicherheitsvorkehrungen gelagert werden muss. Wird beispielsweise das Biogas unter (sehr) hohem Druck gespeichert, kann dadurch zwar das Speichervolumen des Biogases deutlich reduziert werden, allerdings ist dies in der Regel mit einem hohen Energieverlust und/oder einem großen technischen Aufwand verbunden. Wird umgekehrt herum das Biogas bei niedrigem Druck gespeichert, ist der technische Aufwand hierfür zwar geringer, allerdings sind deutlich größere Speichervolumina der entsprechenden Vorrichtungen erforderlich. Außerdem sind die entsprechenden Biogasreaktoren sowie gegebenenfalls Vorratsbehälter ständig der Gefahr von Gasleckagen ausgesetzt.

Im Gegensatz dazu wird im erfindungsgemäßen Verfahren Energie in Form von Wasserstoff gespeichert bzw. gelagert, wobei vorzugsweise in einem vorgelagerten Verfahrensschritt auf derselben Anlage zunächst Wasserstoff erzeugt wird, der wiederum selbst ein brennbares Gas darstellt. Allerdings wird der Wasserstoff durch Inkontaktbringen mit einem geeigneten Träger auf sichere Art und Weise gespeichert. Vorzugsweise reagiert der Wasserstoff mit dem Träger in einer chemischen Reaktion, insbesondere in einer Hydrierung, chemisch ab, sodass der Wasserstoff in modifizierter Form sicher gespeichert bzw. gelagert werden kann. Dies bedeutet, dass insbesondere aufgrund der chemischen Reaktion des Wasserstoffs eine Lagerung unter Normaldruck im erfindungsgemäßen Verfahren möglich ist. Die Speicherung des Wasserstoffs an oder im Träger ist jedoch reversibel, sodass im Bedarfsfall, in der Regel nach einer längeren Lagerung, der Wasserstoff wieder problemlos aus dem entsprechenden Träger freigesetzt werden kann. Der freigesetzte Wasserstoff kann anschließend unmittelbar zur Strom- oder gegebenenfalls zur Wärmeerzeugung eingesetzt werden.

Demzufolge kann mit dem erfindungsgemäßen Verfahren Energie über einen längeren Zeitraum sicher zwischengespeichert werden. Dieser Vorteil kann sehr anschaulich anhand der nachfolgenden Fallkonstellation verdeutlicht werden. Beispielsweise wird in günstigen Zeiten ein Überschuss an Energie in Form von elektrischem Strom erzeugt. Denkbar sind hier saisonal verteilte windreiche Tage im Zusammenhang mit der Erzeugung von elektrischem Strom durch Windenergie bzw. sonnenreiche Tage, insbesondere während des Sommerhalbjahres, zur Erzeugung von elektrischem Strom, beispielsweise über Sonnenkollektoren. Der jeweilige Überschuss an Energie, insbesondere an elektrischem Strom, kann erfindungsgemäß in Wasserstoff umgesetzt werden, der wiederum durch Inkontaktbringen mit einem Träger sicher gespeichert sowie über einen längeren Zeitraum gelagert werden kann. Im Bedarfsfall, also bei einem plötzlich eintretenden erhöhten bzw. kontinuierlichen Bedarf an elektrischer Energie, kann der so zwischengespeicherte Wasserstoff bzw. die zwischengespeicherte Energie wieder in elektrischen Strom oder gegebenenfalls in Wärme rückgewandelt bzw. umgewandelt werden.

Ein weiterer Vorteil des erfindungsgemäßen Verfahrens ist darin zu sehen, dass ein Wärmeverbundsystem zusätzlich durchgeführt werden kann. In der Regel ist die Speicherung des Wasserstoffs in oder an dem Träger (T1) eine exotherme Reaktion, bei der somit Energie freigesetzt wird. Dies ist insbesondere der Fall, wenn die Speicherung des Wasserstoffs im Rahmen einer Hydrierung durchgeführt wird. Im Gegensatz dazu ist die Freisetzung des Wasserstoffs aus dem Träger (T2) in der Regel ein endothermer Vorgang, insbesondere wenn dies als Dehydrierung durchgeführt wird. Demzufolge kann die gegebenenfalls zwischengespeicherte Wärme, die bei der exothermen Reaktion anfällt, für die endotherme Rückreaktion bei der Freisetzung des Wasserstoffs wiederverwendet werden. Die Zwischenspeicherung von Wärme kann vorzugsweise in Erdwärmespeichern durchgeführt werden. Die Variante der zwischengespeicherten Wärme ist insbesondere dann vorteilhaft, wenn zwischen der Speicherung des Wasserstoffs durch Inkontaktbringen mit einem Träger (T1) und der nachfolgenden (reversiblen) Freisetzung von Wasserstoff kein allzu langer Zeitraum liegt. So kommen in der Praxis durchaus Fälle vor, wo an günstigen Tagen ein großer Energieüberschuss in Strom bereitsteht, kurz darauf, beispielsweise nach ein oder zwei Tagen oder Wochen, jedoch plötzlich ein Stromengpass vorhanden ist, sodass ein plötzlicher Strombedarf eintritt. Je länger die Zeitspanne zwischen Wasserstoffspeicherung und Wasserstofffreisetzung jedoch beträgt, umso unwirtschaftlicher wird die Variante der Wärmezwischenspeicherung, weil infolge der Zwischenspeicherung mit der Zeit auch Energie/Wärme verloren geht. Denkbar ist in diesem Zusammenhang auch ein plötzlich auftretender Betrieb eines Notfallgenerators zur Stromerzeugung.

Ebenso kann die bei der nachfolgenden Stromerzeugung freiwerdende Wärme zur Durchführung der in der Regel endothermen Freisetzung des Wasserstoffs eingesetzt werden. Außerdem kann die bei der in der Regel exotherm verlaufenden Speicherung des Wasserstoffs freiwerdende Wärme auch sofort im Rahmen des landwirtschaftlichen Betriebs, beispielsweise zur Beheizung von Gewächshäusern, verwendet werden. Weiterhin kann in vorteilhafter Weise die freigesetzte Wärme in einer parallel oder zeitversetzt durchgeführten Erzeugung von Biogas eingesetzt werden.

Nachfolgend wird das erfindungsgemäße Verfahren zur Umrüstung einer Biogasanlage in eine Anlage zum Lagern von Wasserstoff näher erläutert.

Ein erster Gegenstand der vorliegenden Erfindung ist ein Verfahren zur Umrüstung einer Biogasanlage in eine Anlage zum Lagern von Wasserstoff, umfassend die nachfolgenden Schritte a) bis c), wobei der Schritt b) optional durchgeführt wird.

Biogasanlagen als solche sind, wie eingangs bereits erwähnt, dem Fachmann bekannt. In der Regel weisen Biogasanlagen (unter anderem) ein oder mehrere Fermenter, Vorrats- oder Lagerbehälter, Vorrichtungen zur Erzeugung von Strom, insbesondere Gasmotoren, sowie sonstige Leitungen auf.

Zentraler Bestandteil einer solchen Biogasanlage ist (bezogen auf die vorliegende Erfindung) ein Fermenter, der, wie eingangs bereits erwähnt, in unterschiedlichen Größen und/oder Ausführungen, auch hinsichtlich der in seinem Inneren enthaltenen zusätzlichen Einrichtungselemente, wie Rührwerke oder Heizsysteme, dem Fachmann bekannt ist. Als Fermenter (F1) im Rahmen der vorliegenden Erfindung kann prinzipiell jeder dem Fachmann bekannte Fermenter eingesetzt werden, auch Fermenter, die als Nachgärer bezeichnet bzw. eingesetzt werden.

Vorzugsweise sind die Wände und/oder der Boden im Inneren des Fermenters (F1) aus Beton, Stahl oder Edelstahl, vorzugsweise aus Beton, hergestellt.

Vorzugsweise ist das Dach im Inneren des Fermenters (F1) aus mindestens einem Kunststoff, vorzugsweise aus Polyvinylchlorid (PVC) oder Polyethylen (PE), hergestellt. Weiterhin ist es dabei bevorzugt, dass das PVC in Folienform und/oder der Kunststoff, insbesondere das PE, Garne oder Fasern enthält.

Unter dem Begriff "Umrüstung einer Biogasanlage" wird im Rahmen der vorliegenden Erfindung insbesondere die Umrüstung von mindestens einem Fermenter (F1) verstanden. Darüber hinaus kann die Umrüstung aber auch weitere Elemente einer Biogasanlage, wie Vorrats- oder Lagerbehälter, Vorrichtungen zur Erzeugung von Strom sowie Leitungen betreffen. Vordergründig wird die Umrüstung einer Biogasanlage bzw. vorzugsweise von mindestens einem Fermenter (F1) durch die nachfolgend näher definierten Verfahrensschritte a) sowie gegebenenfalls b) durchgeführt. Darüber hinaus kann die Umrüstung auch weitere Verfahrensschritte umfassen, beispielsweise das Entfernen von Vorrichtungselementen, wie Heizungen und/oder Leitungen, die sich innerhalb des Fermenters (F1) befinden oder die gegebenenfalls auch außerhalb des Fermenters (F1) Bestandteil einer Biogasanlage sind.

Somit ist es prinzipiell auch denkbar, dass der Fermenter (F1) auch nach der Umrüstung weiterhin als Fermenter genutzt wird. Bei dieser Fallkonstellation werden in den Fermenter (F1) entsprechende zusätzliche Vorrichtungsbestandteile oder Elemente eingebaut, die eine Lagerung des mit Wasserstoff beladenen Trägers (T2) gemäß nachfolgend definiertem Schritt c) in dem Fermenter (F1) ermöglichen, ohne dass dabei der bisherige Verwendungszweck im Zusammenhang mit der Herstellung von Biogas eingestellt werden muss. Unter Umständen kann es dabei erforderlich sein, dass weitere Zuleitungen und/oder Ableitungen in den Fermenter (F1) eingebaut werden müssen, um das Einbringen des Trägers (T2) sowie die im Anschluss an die Lagerung erforderliche Entnahme des Trägers (T2) aus dem Fermenter (F1) zu ermöglichen.

Erfindungsgemäß ist es jedoch bevorzugt, dass der Fermenter (F1) so umgebaut/umgerüstet wird, dass der bisherige Verwendungszweck des umgerüsteten Fermenters (F1) im Rahmen der Biogasanlage nicht mehr durchgeführt werden kann. Vorzugsweise können dabei aus dem Fermenter (F1) die in einem Fermenter in der Regel vorhandenen Rührwerkzeuge und die damit verbundenen Bedienelemente oder Leitungen ausgebaut werden. Gegebenenfalls vorhandene Heizelemente können hingegen in dem Fermenter (F1) ohne Probleme belassen werden, da sie auch im Zusammenhang mit der Durchführung des erfindungsgemäßen Verfahrensschritts c) sinnvoll verwendet werden können. Gegebenenfalls können die entsprechenden bisher vorhandenen Zuführ- und/oder Entnahmeelemente in dem Fermenter (F1) zu den für das erfindungsgemäße Verfahren erforderlichen Zwecken umgerüstet werden oder gegebenenfalls ohne Umrüstung eingesetzt werden.

In Verfahrensschritt a) erfolgt das Reinigen des Inneren von mindestens einem Fermenter (F1) der Biogasanlage.

Das Reinigen kann prinzipiell nach allen dem Fachmann bekannten Methoden durchgeführt werden. Die konkrete Durchführung des Reinigungsschritts a) hängt unter anderem auch von der Art und Größe des zu reinigenden Fermenters (F1) ab und/oder von der Dauer sowie den sich im Fermenter befindlichen Substanzen, die zur Erzeugung von Biogas verwendet worden sind. Beispielsweise kann es sich, wie eingangs erwähnt, bei den zur Erzeugung von Biogas verwendeten Substanzen auch um Gülle oder Festmist handeln, die beispielsweise ätzend sein können und somit bei einer langen Betriebsdauer das Innere eines Fermenters beschädigen und/oder kontaminieren können. Ist beispielsweise ein Fermenter nicht lange zur Erzeugung von Biogas in Betrieb gewesen und/oder wurden relativ ungefährliche bzw. nicht oder geringfügig ätzende Stoffe zur Erzeugung von Biogas in dem Fermenter verwendet, kann es erfindungsgemäß ausreichend sein, wenn das Reinigen des Inneren des Fermenters (F1) durch technisch bzw. zeitmäßig relativ einfache Reinigungsschritte durchgeführt wird, wie beispielsweise die nachfolgend definierten Teilschritte a2) oder a4). Diese Schritte können zur Reinigung beispielsweise auch dann ausreichend sein, sofern ein Fermenter (F1) in seinem Inneren vollständig aus Edelstahl hergestellt ist.

War hingegen ein Fermenter (sehr) lange zur Erzeugung von Biogas im Einsatz und/oder wurden als Substanzen relativ ätzende Komponenten, wie Gülle oder Festmist, eingesetzt, ist es hingegen vorteilhaft, wenn der Verfahrensschritt a) die Durchführung des nachfolgend definierten Teilschritts a1) umfasst. Dies ist insbesondere der Fall, wenn Teile des Inneren des Fermenters, insbesondere die Wände und/oder der Boden nicht aus Edelstahl, sondern beispielsweise aus Beton oder herkömmlichem Stahl hergestellt sind. Sofern im Inneren des Fermenters (F1) weitere Vorrichtungselemente, wie Heizungen oder sonstige Leitungen, die zur Erzeugung des Biogases verwendet worden sind, belassen werden, können diese Vorrichtungselemente sinngemäßen Reinigungsschritten unterzogen werden.

Vorzugsweise umfasst der Schritt a) einen Teilschritt a1), wobei vom Inneren des Fermenters (F1) mindestens eine Oberflächenschicht zumindest teilweise entfernt wird. Das Entfernen der Oberflächenschicht kann hierbei nach allen dem Fachmann bekannten Methoden erfolgen. Vorzugsweise erfolgt das Entfernen durch Abschleifen oder Abtragen, insbesondere durch Abschleifen, von mindestens einer Oberflächenschicht. Besonders bevorzugt sind dabei eine abrasive Hochdruckreinigung und/oder die Verwendung von Sandstrahlern. Die Oberfläche im Inneren des Fermenters (F1) kann dabei prinzipiell in beliebigen Dicken und/oder Anzahl von Schichten entfernt, vorzugsweise abgeschliffen, werden. Vorzugsweise erfolgt das Entfernen jedoch nur bis zu einer Schichtdicke von ein paar Millimetern, beispielsweise 1 bis 10 mm, vorzugsweise 2 bis 5 mm. Das Entfernen der Oberflächenschicht erfolgt vorzugsweise vollständig, insbesondere von den Wänden und/oder dem Boden im Inneren des Fermenters (F1). Weiterhin ist es bevorzugt, dass Teilschritt a) an den Bereichen im Inneren des Fermenters (F1) durchgeführt wird, die aus Beton, Stahl oder gegebenenfalls aus Stahlbeton hergestellt worden sind. Sofern das Innere des Fermenters aus Stahlbeton hergestellt ist, wird Teilschritt a) nur soweit durchgeführt, dass die Armierung des Stahlbetons und/oder die die Armierung enthaltende Schicht des Stahlbetons nicht entfernt wird.

Weiterhin kann Schritt a) einen Teilschritt a2) umfassen, wobei im Inneren des Fermenters (F1) mindestens eine alkalische Wäsche durchgeführt wird. Die Durchführung der alkalischen Wäsche erfolgt prinzipiell nach dem Fachmann bekannten Methoden. Der Teilschritt a2) kann gegebenenfalls auch mehrfach wiederholt werden und/oder durch eine Wäsche im neutralen Bereich, also unter Verwendung von Wasser ohne alkalische Zusätze, unterbrochen werden. Zur alkalischen Wäsche können alle dem Fachmann bekannten Chemikalien verwendet werden, die einen pH-Wert im Bereich > 7, bevorzugt 8 bis 14, insbesondere 9 bis 13, ermöglichen. Beispielsweise können hierfür auch Natronlauge oder sonstige Basen verwendet werden, deren genauer pH-Wert durch die Zugabe von Wasser oder gegebenenfalls von Puffern eingestellt wird.

Weiterhin kann Schritt a) einen Teilschritt a3) umfassen, wobei aus dem Inneren des Fermenters (F1) sowie gegebenenfalls aus seinen Zuleitungen alle Substanzen, die sich infolge der Erzeugung von Biogas im Inneren des Fermenters (F1) oder gegebenenfalls in seinen Zuleitungen befinden, entfernt werden. Vorzugsweise erfolgt das Entfernen gemäß Teilschritt a3) durch Ablassen, vorzugsweise durch Absaugen, der Substanzen. Der Teilschritt a3) wird erfindungsgemäß vorzugsweise immer dann durchgeführt, sofern noch Substanzen von der zuvor durchgeführten Erzeugung von Biogas im Inneren des Fermenters bzw. seinen Zuleitungen vorhanden sind.

Weiterhin kann Schritt a) auch einen Teilschritt a4) umfassen, in dem das Innere des Fermenters (F1) mit Wasser oder einem wässrigen Reinigungsmittel gespült wird. Im Anschluss daran kann gegebenenfalls noch ein Trocknungsschritt durchgeführt werden. Vorzugsweise umfasst Schritt a) einen Teilschritt a4), wobei im Anschluss an mindestens einen der Teilschritte a1) bis a3) mit Wasser oder einem wässrigen Reinigungsmittel gespült sowie gegebenenfalls danach ein Trocknungsschritt durchgeführt wird.

Weiterhin ist es bevorzugt, dass
i) von Schritt a) die Teilschritte a1) und a2) durchgeführt werden, oder
ii) von Schritt a) die Teilschritte a1), gefolgt von a2) und gefolgt von a4) durchgeführt werden, wobei gegebenenfalls vor Durchführung des Teilschritts a1) der Teilschritt a3) durchgeführt wird, oder
iii) der Teilschritt a3) zusätzlich vor einem der Teilschritte a1), a2) oder a4), vorzugsweise vor Teilschritt a1), durchgeführt wird.

Weiterhin ist es bevorzugt, dass zur Reinigung der Decke des Fermenters (F1) der Teilschritt a2) und/oder der Teilschritt a4) durchgeführt wird. Ebenso ist es bevorzugt, dass die Reinigung einer Zuleitung des Fermenters (F1) bis zur ersten Absperrvorrichtung der Zuleitung erfolgt und/oder eine Zuleitung des Fermenters (F1) im Inneren des Fermenters (F1) verschlossen wird, vorzugsweise durch Anbringen eines Stopfens. Ebenso ist es bevorzugt, dass ein im Inneren des Fermenters (F1) vorhandenes Rührwerkzeug vor Durchführung von einem der Teilschritte a1), a2) oder a4) und/oder nach Durchführung des Teilschritts a3) ausgebaut wird.

Im optional durchgeführten Verfahrensschritt b) erfolgt gegebenenfalls das Anbringen von mindestens einer Schutzauskleidung im Inneren des gereinigten Fermenters (F1). Das Anbringen der Schutzauskleidung kann prinzipiell nach allen dem Fachmann bekannten Methoden erfolgen, beispielsweise durch Aufkleben oder eine mechanische Befestigung wie Festnageln, Anschrauben oder Verspannen. Hierfür geeignete Hilfsmittel, wie Klebstoffe, Schrauben oder Nägel sind dem Fachmann bekannt. Sofern Klebstoffe verwendet werden, können hierfür prinzipiell alle bekannten Klebstoffe verwendet werden, wie anorganische oder organische Klebstoffe, beispielsweise auf Metalloxiden basierende Kleber oder Polyurethanklebstoffe.

Im Rahmen der vorliegenden Erfindung ist es bevorzugt, dass Schritt b) durchgeführt wird. Schritt b) wird insbesondere dann durchgeführt, wenn das Innere des Fermenters (F1) vollständig oder zumindest teilweise, vorzugsweise vollständig, nicht aus Edelstahl hergestellt ist. Besonders bevorzugt wird Schritt b) dann durchgeführt, sofern die Wände und/oder der Boden im Inneren des Fermenters (F1) aus Beton hergestellt sind. Vorzugsweise wird Schritt b) hingegen nicht durchgeführt, sofern die Wände und/oder der Boden im Inneren des Fermenters (F1) aus Edelstahl hergestellt sind. Weiterhin ist es bevorzugt, dass ein im Inneren des Fermenters (F1) vorhandenes Rührwerkzeug vor Durchführung von Schritt c) oder gegebenenfalls vor Durchführung von Schritt b) ausgebaut wird.

Als Schutzauskleidung können prinzipiell alle dem Fachmann bekannten Schutzauskleidungen verwendet werden, beispielsweise in Form von Folien, als sogenannte Liner oder als Beschichtungen aus Epoxidharzen. Die Schutzauskleidung kann im Inneren des (gereinigten) Fermenters (F1) so angebracht werden, dass sie vollständig oder zumindest teilweise das komplette Innere des Fermenters (F1) bedeckt. Vorzugsweise werden diejenigen Bereiche des (gereinigten) Fermenters (F1) mit Schutzauskleidung bedeckt, die aus Beton oder Stahl sowie Stahlbeton hergestellt worden sind. Ist beispielsweise das Dach im Inneren des Fermenters (F1) aus Kunststoff hergestellt, ist es nicht erforderlich, solche aus Kunststoff hergestellten Bereiche mit einer zusätzlichen Schutzauskleidung zu versehen. Gegebenenfalls kann dies aber trotzdem gemacht werden. Vorzugsweise bedeckt die Schutzauskleidung den Boden und/oder die Wände im Inneren des gereinigten Fermenters (F1) vollständig oder zumindest größtenteils.

Weiterhin ist es bevorzugt, dass die Schutzauskleidung aus mindestens einem Kunststoff hergestellt ist, vorzugsweise ist der Kunststoff ausgewählt aus einem Polyalkylen, einem Epoxidharz, einem Polyurethan und/oder einer Plastikfolie, mehr bevorzugt ist der Kunststoff ausgewählt aus einem Polyethylen (PE), einem HD (high density)-Polyethylen (HD-PE), einem Polypropylen (PP) und/oder einem Polyurethan (PU).

Weiterhin ist es bevorzugt, dass die Schutzauskleidung mit Glasfasern verstärkt ist, vorzugsweise ist die Schutzauskleidung aus mindestens einem glasfaserverstärkten Kunststoff hergestellt. Die Schutzauskleidung kann gegebenenfalls mit Glasfasern verstärkt sein. Sofern dies der Fall ist, ist die Schutzauskleidung bevorzugt aus mindestens einem glasfaserverstärkten Kunststoff hergestellt.

Die Schutzauskleidung selbst kann wiederum genau eine Schicht aufweisen, bevorzugt kann sie auch zwei oder mehrere Schichten umfassen. Sofern die Schutzauskleidung einschichtig ist, erfolgt das Anbringen der Schutzauskleidung im Inneren des gereinigten Fermenters (F1) vorzugsweise durch Verkleben. Sofern sie zwei- oder mehrschichtig ist, erfolgt das Anbringen vorzugsweise im Rahmen einer mechanischen Befestigung, insbesondere durch Verspannen. Prinzipiell ist es denkbar, dass im Inneren des Fermenters (F1) in unterschiedlichen Bereichen unterschiedliche Arten von Schutzauskleidungen angebracht werden. Gegebenenfalls können auch mehrere Schutzauskleidungen übereinander angebracht werden. Vorzugsweise wird im Inneren des Fermenters (F1) eine Art von Schutzauskleidung einheitlich verwendet, wobei die Schutzauskleidung gegebenenfalls auch aus mehreren Schichten aufgebaut sein kann.

In einer bevorzugten Ausführungsform der vorliegenden Erfindung ist die Schutzauskleidung aus mindestens zwei Schichten aufgebaut und gegebenenfalls mit einer zusätzlichen Druckkontrolleinrichtung versehen, vorzugsweise befindet sich ein Gas, insbesondere Luft, zwischen zwei Schichten der Schutzauskleidung. Weiterhin ist es bevorzugt, dass das Gas unter Druck ist. Der Druck kann beliebige Werte annehmen, vorzugsweise beträgt der Druck des Gases 1,5 bis 10 bara (bar absolut). Der Druck kann über eine permanente Druckmessung überwacht werden. So kann eine potentielle Leckage der inneren oder äußeren Schutzauskleidung frühzeitig in Form eines Druckabfalls erkannt werden, ohne dass das Trägermedium aus dem Behälter lecken kann.

In einer weiteren Ausführungsform der vorliegenden Erfindung wird eine mindestens zwei Schichten aufweisende Schutzauskleidung, die eine Schicht aus Polyethylen und eine Schicht aus HD-Polyethylen umfasst, so an die Wände und/oder den Boden im Inneren des Fermenters (F1) angebracht, dass die Schicht aus HD-Polyethylen sich auf der von den Wänden und/oder dem Boden abgewandten Seite der Schutzauskleidung im Inneren des Fermenters (F1) befindet.

In einer weiteren Ausführungsform sind die Wände und/oder der Boden im Inneren des Fermenters (F1) aus Beton hergestellt und eine Schutzauskleidung, die aus einem Polypropylen oder einem Polyurethan hergestellt ist, wird so auf den Beton aufgebracht, dass die Schicht der Schutzauskleidung, die aus einem Polypropylen oder einem Polyurethan hergestellt ist, direkt an den Beton angebracht ist. Sofern bei dieser Ausführungsform die Schutzauskleidung durch Kleben angebracht wird, bedeutet dies, dass sich der Klebstoff zwischen Schutzauskleidung und Boden oder Wand befindet.

In einer Ausführungsform der vorliegenden Erfindung ist es weiterhin bevorzugt, dass in das Innere des gereinigten Fermenters (F1) zwei räumlich getrennte Bereiche eingebaut werden, wobei vorzugsweise die räumliche Trennung durch Einbau einer schwimmenden Trennwand in horizontaler Richtung oder durch Einbau einer Trennwand in vertikaler Richtung im Inneren des Fermenters (F1) erfolgt, wobei die Trennwand gegebenenfalls mit mindestens einer Schutzauskleidung versehen ist, besonders bevorzugt erfolgt die räumliche Trennung durch Einbau einer Trennwand in vertikaler Richtung im Inneren des Fermenters (F1), wobei die Trennwand gegebenenfalls mit mindestens einer Schutzauskleidung versehen ist.

Diese Ausführungsform der vorliegenden Erfindung wird im Anschluss an die Durchführung von Schritt a) sowie gegebenenfalls nach Durchführung von Schritt b) durchgeführt. Vorzugsweise wird diese Ausführungsform erst im Anschluss an die Durchführung von Schritt b) durchgeführt. Dies ist insbesondere dann der Fall, sofern der Einbau einer Trennwand in vertikaler Richtung erfolgt. Diese Ausführungsform kann insbesondere dann durchgeführt werden, wenn, wie vorstehend bereits erwähnt, der Fermenter (F1) weiterhin im Rahmen seiner ursprünglichen Nutzung zur Verwendung zur Herstellung von Biogas eingesetzt wird. Weiterhin ist diese Ausführungsform bevorzugt, sofern der Fermenter (F1) nicht mehr zur Erzeugung von Biogas benutzt werden soll, stattdessen im Fermenter (F1), wie nachfolgend näher ausgeführt, sowohl die Lagerung des mit Wasserstoff beladenen Trägers (T2) als auch die Lagerung des Trägers (T1) durchgeführt werden soll. Sofern in den Fermenter (F1) eine schwimmende Trennwand in horizontaler Richtung eingebaut wird, ist es bevorzugt, dass der mit Wasserstoff beladene Träger (T2) unterhalb der schwimmenden Trennwand gelagert wird.

In Verfahrensschritt c) erfolgt das Einbringen und Lagern eines mit Wasserstoff beladenen Trägers (T2) in den gegebenenfalls im Inneren mit einer Schutzauskleidung versehenen gereinigten Fermenter (F1).

Dies bedeutet, dass Verfahrensschritt c) mit einem "nur" gereinigten Fermenter (F1) durchgeführt wird, sofern der vorstehend erwähnte optionale Verfahrensschritt b) nicht durchgeführt worden ist. Sofern im Rahmen der vorliegenden Erfindung Verfahrensschritt b) jedoch vorzugsweise durchgeführt wird, wird in Verfahrensschritt c) ein im Inneren mit (mindestens) einer Schutzauskleidung versehener, gereinigter Fermenter (F1) eingesetzt.

Das Einbringen des mit Wasserstoff beladenen Trägers (T2) in den Fermenter (F1) erfolgt in der Regel unter Verwendung von bereits im Fermenter (F1) enthaltenen Vorrichtungselementen, wie Leitungen oder Öffnungen, die, wie vorstehend bereits erwähnt, vorzugsweise auch einer (zusätzlichen) Reinigung unterzogen worden sind. Gegebenenfalls ist es auch denkbar, dass zum Einbringen des mit Wasserstoff beladenen Trägers (T2) zusätzliche Vorrichtungselemente in den Fermenter (F1) eingebaut werden. Das Einbringen als solches ist dem Fachmann bekannt, beispielsweise kann dies durch Pumpen und/oder der Anwendung von Druck erfolgen.

Die Lagerung des mit Wasserstoff beladenen Trägers (T2) kann prinzipiell für beliebige Zeiträume stattfinden. In der Regel erfolgt die Lagerung für mindestens mehrere Tage, sie kann aber auch mehrere Wochen, Monate oder gar Jahre betragen. Genauso ist es aber auch denkbar, dass die Lagerung nur für wenige Minuten oder Stunden oder für einen Tag durchgeführt wird. Vorzugsweise erfolgt die Lagerung für mindestens zwei Tage und/oder maximal sechs Monate.

Mit Wasserstoff beladene Träger (T2) als solche sind dem Fachmann prinzipiell bekannt. Im Träger (T2) ist der Wasserstoff in der Regel chemisch oder physisorptiv, vorzugsweise chemisch, an den Träger gebunden. Wie nachfolgend im Zusammenhang mit dem optionalen Verfahrensschritt d) detailliert ausgeführt, wird erfindungsgemäß der mit Wasserstoff beladene Träger (T2) vorzugsweise aus einem Träger (T1) hergestellt, insbesondere durch Hydrierung des Trägers (T1) mit Wasserstoff.

Die nachfolgend beschriebenen optionalen Verfahrensschritte d) sowie gegebenenfalls e) können zeitgleich, vor oder auch nach den vorstehend beschriebenen Verfahrensschritten a) und b) durchgeführt werden. Die Durchführung der Verfahrensschritte a) und b) einerseits sowie d) und e) andererseits ist prinzipiell vollkommen unabhängig voneinander. Insbesondere bei den Verfahrensschritten d) und e) handelt es sich um Verfahrensschritte, die auch mehrmals und/oder kontinuierlich durchgeführt werden können, während hingegen die Verfahrensschritte a) und b) vorzugsweise nur einmal im Rahmen des konkreten Umrüstungsvorganges einer Biogasanlage bzw. eines Fermenters durchgeführt werden. Die Anzahl sowie konkrete Ausgestaltung der Durchführung der Verfahrensschritte d) und e) hängt hingegen mehr von der Art und Anzahl der Durchführung der dem Verfahrensschritt c) nachgelagerten Verfahrensschritte f) sowie g) ab, also der Erzeugung von Strom unter Verwendung des in Verfahrensschritt c) gelagerten Wasserstoffs.

Im erfindungsgemäßen optionalen Verfahrensschritt d) erfolgt ein Inkontaktbringen eines Trägers (T1) mit Wasserstoff zur Speicherung des Wasserstoffs unter Erhalt des mit Wasserstoff beladenen Trägers (T2). Verfahrensschritt d) wird vor Verfahrensschritt c) durchgeführt.

Als Träger (T1) kann prinzipiell jeder beliebige, dem Fachmann bekannte Träger verwendet werden, der dazu geeignet ist, Wasserstoff in irgendeiner Form zu speichern. Weiterhin ist erfindungsgemäß der Träger (T1) dazu in der Lage, den Wasserstoff reversibel zu speichern. Dies bedeutet, dass aus dem mit Wasserstoff beladenen Träger (T2), der durch Inkontaktbringen des Wasserstoffs mit dem Träger (T1) erhalten wird, der Wasserstoff zu einem späteren Zeitpunkt (also nach der Lagerung gemäß Verfahrensschritt c)) wieder freigesetzt werden kann und dabei der ursprünglich eingesetzte Träger (T1) wiedergewonnen wird.

Das Speichern des Wasserstoffs kann beispielsweise so durchgeführt werden, dass Wasserstoff chemisch oder physisorptiv, vorzugsweise chemisch, an den Träger (T1) gebunden wird. Im Rahmen der vorliegenden Erfindung ist es jedoch erforderlich, dass das Speichern des Wasserstoffs reversibel möglich ist. Insbesondere Kohlendioxid, das chemisch mit Wasserstoff unter Erhalt von Methan reagieren kann, ist kein Träger (T1) im Sinne der vorliegenden Erfindung, weil aus Methan nicht direkt und/oder auf einfachem Wege reversibel Wasserstoff freigesetzt sowie Kohlendioxid wiedergewonnen werden kann.

Folglich ist Methan auch kein mit Wasserstoff beladener Träger (T2) im Sinne der vorliegenden Erfindung.

Eine chemische Bindung des Wasserstoffs an den Träger (T1) kann beispielsweise dadurch erfolgen, dass der Träger (T1) ein Metallhydridspeicher ist, der ein aus einer Metalllegierung hergestelltes Material enthält, das Wasserstoff aufnimmt und chemisch bindet. Alternativ kann der Träger (T1) auch eine, vorzugsweise flüssige, organische Verbindung sein. Solche organischen Verbindungen werden auch als Wasserstoffträger oder LOHC (englisch für: liquid organic hydrogen carriers) bzw. organische Hydride bezeichnet. Solche Wasserstoffträger (LOHC) sind dem Fachmann bekannt und beispielsweise in DE-B 10 2013 223 589, EP-A 1 475 349 und DE-A 10 2012 005 023 beschrieben.

Diese Form der Wasserstoffspeicherung unter Verwendung der LOHCs hat den besonderen Vorteil, dass der LOHC-Träger (T1) in der Regel unter den verwendeten Prozessbedingungen in flüssiger Form vorliegt. Die physiochemischen Eigenschaften eines solchen LOHC-Trägers haben hohe Ähnlichkeit zu herkömmlichen flüssigen Kraftstoffen, sodass Pumpen zum Transport oder Behälter zur Lagerung aus dem Bereich der Kunststoff- und Printstofflogistik genutzt werden können. Die Wasserstoffspeicherung in chemisch gebundener Form in einer organischen Flüssigkeit wie LOHC erlaubt eine drucklose Lagerung bei Normalbedingungen über große Zeiträume ohne signifikanten Wasserstoffverlust. Sofern es sich bei dem Träger (T1) um einen LOHC-Träger handelt, liegt der Träger zur Aufnahme von Wasserstoff in seiner vollständig oder zumindest teilweise dehydrierten Form vor. Vorzugsweise handelt es sich dabei um die vollständig dehydrierte Form. Eine zumindest teilweise dehydrierte Form kann dann vorliegen, wenn beispielsweise eine vollständig dehydrierte Form eines solchen Trägers über zwei oder mehrere Stellen verfügt, an denen das entsprechende Molekül hydriert werden kann, und mindestens eine dieser zur Hydrierung geeigneten Stellen bereits in hydrierter Form vorliegt, aber noch mindestens eine Stelle in dehydrierter (nicht hydrierter) Form vorliegt. Beispiele hierfür sind unter anderem aromatische Verbindungen, insbesondere polyzyklische aromatische Verbindungen mit einem konjugierten π-Elektronensystem.

Das Inkontaktbringen des Trägers (T1) mit Wasserstoff kann prinzipiell nach beliebigen, dem Fachmann bekannten Methoden durchgeführt werden, beispielsweise indem Wasserstoff über den Träger (T1) übergeleitet wird, durch den Träger (T1) durchgeleitet wird oder auf den Träger (T1) aufgepresst wird.

Durch das Inkontaktbringen des Trägers (T1) mit Wasserstoff wird in Schritt d) des erfindungsgemäßen Verfahrens somit der mit Wasserstoff beladene Träger (T2) erhalten. Sowohl der Träger (T1) als auch der durch Inkontaktbringen mit Wasserstoff erhaltene mit Wasserstoff beladene Träger (T2) können als Gemisch von zwei oder mehreren Trägern (T1) und/oder (T2) vorliegen. Sofern der Wasserstoff chemisch an den Träger (T1) gebunden wird, handelt es sich dabei um eine vollständige oder zumindest teilweise Hydrierung des entsprechenden Trägers (T1), sodass sich der mit Wasserstoff beladene Träger (T2) chemisch vom entsprechenden unbeladenen Träger (T1) unterscheidet. Vorzugsweise handelt es sich dabei um eine vollständige Hydrierung des Trägers (T1) mit Wasserstoff unter Erhalt des mit Wasserstoff beladenen Trägers (T2).

Das Verhältnis der Träger (T1) und (T2) im erfindungsgemäßen Verfahren gemäß Schritt d) wird nachfolgend an dem aus dem Stand der Technik bekannten LOHC-Träger Toluol erläutert. Toluol enthält einen Benzolring und somit drei π-Elektronenpaare. Toluol stellt also einen Träger (T1) dar, der in seiner vollständig dehydrierten Form vorliegt. Durch Inkontaktbringen des Toluols mit Wasserstoff wird erfindungsgemäß eine zumindest teilweise oder vorzugsweise vollständige Hydrierung von Toluol zu Methylcyclohexan als Träger (T2) erzielt. Sofern die vollständige Hydrierung von Toluol durchgeführt wird, werden also alle drei π-Elektronenpaare des Toluols hydriert, sodass Methylcyclohexan die vollständig hydrierte Form des Toluols darstellt. Sofern nur ein oder zwei der π-Elektronenpaare des Toluols hydriert worden sind, handelt es sich nur um eine teilweise Hydrierung. Solche zumindest teilweise hydrierten Verbindungen können sowohl als Träger (T1) oder als Träger (T2) erfindungsgemäß eingesetzt werden.

Normalerweise ist der Träger (T1) eine ungesättigte Verbindung, bevorzugt eine Verbindung mit mindestens einer Kohlenstoff-Kohlenstoff-Doppelbindung, mehr bevorzugt eine aromatische oder hetero-aromatische Verbindung, noch mehr bevorzugt ein N-Alkylcarbazol, Toluol, Dibenzyltoluol, Benzyltoluol, Naphthalin oder ein Azaborin, besonders bevorzugt Dibenzyltoluol.

Bei Verbindungen mit mindestens einer Kohlenstoff-Kohlenstoff-Doppelbindung (C-C-Doppelbindung) kann es sich beispielsweise um ein Olefin, einen Aromaten oder einen Heteroaromaten handeln. Solche Verbindungen sind dem Fachmann prinzipiell bekannt. Diese Verbindungen können gegebenenfalls substituiert sein, beispielsweise durch eine oder mehrere Alkylgruppen von beliebiger Kettenlänge, wie Methyl, Ethyl, Propyl oder durch sonstige funktionelle Gruppen, wie Halogen, Amino oder Carboxyl. Heteroaromatische Verbindungen weisen als Heteroatom mindestens ein Heteroatom auf, das vorzugsweise Sauerstoff, Stickstoff und/oder Schwefel ist. Aromatische oder heteroaromatische Verbindungen können monozyklisch, bizyklisch oder gegebenenfalls polyzyklisch sein. Gegebenenfalls können die entsprechenden Zyklen (Ringe) zumindest teilweise auch in hydrierter Form vorliegen. Ebenso ist es denkbar, dass beispielsweise bei einer bizyklischen Verbindung einer der beiden Zyklen vollständig hydriert ist.

Sofern als Träger (T1) ein N-Alkylcarbazol eingesetzt wird, kann der an das Stickstoffatom gebundene Alkylrest eine beliebige Kettenlänge annehmen, beispielsweise kann es sich dabei um Methyl, Ethyl, Propyl, Isopropyl oder Butyl sowie gegebenenfalls Mischungen davon handeln. Vorzugsweise handelt es sich dabei um N-Ethylcarbazol.

Demzufolge wird im erfindungsgemäßen Verfahren in Schritt d) vorzugsweise die entsprechende teilweise oder insbesondere vollständig hydrierte Form der vorstehend definierten konkreten Träger (T1) als mit Wasserstoff beladener Träger (T2) erhalten.

Prinzipiell ist es möglich, dass Schritt d) im gleichen Fermenter (F1), der gereinigt und gegebenenfalls im Inneren mit einer Schutzauskleidung versehen ist, durchgeführt wird wie der vorstehend definierte Verfahrensschritt c). Dies kann erfolgen, wenn beispielsweise in den Fermenter (F1) eine spezielle Einheit/Vorrichtungsbestandteil integriert wird, der die Durchführung des Verfahrensschritts d) getrennt von der Durchführung des Verfahrensschritts c), also insbesondere der Lagerung des mit Wasserstoff beladenen Trägers (T2), ermöglicht. Entsprechende Einheiten oder Einbaumaßnahmen sind dem Fachmann bekannt. Vorzugsweise wird jedoch im erfindungsgemäßen Verfahren Schritt d) in einer separaten Vorrichtung (V3) durchgeführt, die vorzugsweise ein chemischer Reaktor ist.

Vorzugsweise wird Schritt d) in einer druckstabilen Vorrichtung, insbesondere in einem druckstabilen chemischen Reaktor, durchgeführt. Weiterhin ist es bevorzugt, dass Schritt d) bei einer Temperatur zwischen 50°C und 400°C, insbesondere zwischen 120°C und 300°C, insbesondere zwischen 150°C und 280°C durchgeführt wird. Die Hydrierung, also das Beladen des Trägers (T1) mit Wasserstoff, findet vorzugsweise bei einem Verfahrensdruck von 2 bar bis 200 bar, insbesondere bei 10 bar bis 100 bar, statt. Weiterhin ist es bevorzugt, dass Schritt d) in Gegenwart eines metallhaltigen Katalysators durchgeführt wird. Als Katalysatoren für die Beladung des LOHC-Trägers (T1) eignen sich insbesondere solche, die das Element Ruthenium und/oder Nickel aufweisen. Es sind auch Katalysatoren möglich, die andere Elemente oder zusätzliche Elemente neben Ruthenium und/oder Nickel aufweisen. Wesentlich sind solche Elemente, die Wasserstoff anlagern und auf LOHC-Träger (T1) übertragen können. Neben Ruthenium und/oder Nickel sind insbesondere Metalle wie Chrom, Eisen, Kobalt, Kupfer, Iridium, Palladium oder Platin als Katalysatoren möglich.

Im erfindungsgemäßen Verfahren ist es bevorzugt, dass vor (dem optionalen) Schritt d) ein Schritt e) durchgeführt wird. Gemäß des optionalen erfindungsgemäßen Schritts e) erfolgt die Erzeugung von Wasserstoff in einer Vorrichtung (V6), vorzugsweise in einem Elektrolyseur, unter Verwendung von Wasser und elektrischem Strom. Die Durchführung des Verfahrensschritts e) als solchem ist dem Fachmann bekannt. Geeignete Elektolyseure sind beispielsweise in DE-B 10 2013 223 589 oder DE-A 10 2012 005 023 beschrieben. Hierbei kann es sich beispielsweise um einen sogenannten PEM-Elektrolyseur (Polymerelektrolytmembran) oder um einen SOEC-Elektrolyseur (solid oxide electolysis cell) sowie um einen Hochtemperatur-Elektrolyseur handeln. Letztendlich ist als Vorrichtung (V6) jede Art von Vorrichtung geeignet, in der die Zerlegung von Wasser in Wasserstoff oder Sauerstoff bei gleichzeitiger Einspeisung von elektrischem Strom durchgeführt werden kann. In der Vorrichtung (V6) sind in der Regel zwei Elektroden vorhanden. Bei dem eingesetzten Wasser handelt es sich normalerweise um leitfähiges Wasser, das gegebenenfalls weitere Zusätze enthalten kann, um die Wasserelektrolyse zu ermöglichen.

Der in Schritt e) verwendete elektrische Strom kann prinzipiell aus beliebigen Quellen stammen. So ist es denkbar, dass der elektrische Strom einem separaten Netz (Verbrauchernetz) entnommen wird. Vorzugsweise handelt es sich jedoch bei dem eingesetzten Strom gemäß Schritt e) um regenerativ hergestellten elektrischen Strom (Elektrizität). Dieser regenerativ hergestellte elektrische Strom wird vorzugsweise direkt oder in unmittelbarer Nähe der Durchführung des erfindungsgemäßen Verfahrens erzeugt. Der elektrische Strom kann beispielsweise aus Wasserkraft, Windenergie, solarer Strahlung (Sonnenenergie), Erdwärme (Geothermie), Biogas, Bioethanol, Holz oder durch die Gezeiten gewonnen werden. Die zur Gewinnung des elektrischen Stroms notwendigen Maschinen und Apparate sind ebenfalls bekannt. Es kann sich dabei um Wasserturbinen, Windanlagen, Fotovoltaik, Solarthermie, Verbrennungsmaschinen oder Turbinen, bei deren Brennstoff es sich um Biomasse handelt, stammen. Vorzugsweise wird der in Schritt e) des erfindungsgemäßen Verfahrens eingesetzte elektrische Strom durch Wasserkraft, Windenergie oder solare Strahlung erzeugt. Da dieser elektrische Strom prinzipiell auch aus von nachwachsenden Rohstoffen gewonnener Biomasse, insbesondere Biogas, stammen kann, ist es auch denkbar, dass das in einer Ausführungsform der vorliegenden Erfindung parallel oder zeitversetzt hergestellte Biogas in einem nachfolgenden Schritt verstromt wird und dieser Strom, für den aktuell keine Verwendung besteht, im Rahmen des erfindungsgemäßen Verfahrens in Wasserstoff umgewandelt wird, der erfindungsgemäß gespeichert und gelagert wird.

Wie vorstehend bereits erwähnt, wird in Schritt e) (in der Regel) neben Wasserstoff zusätzlich Sauerstoff erzeugt. Vorzugsweise wird in Schritt c) zusätzlich Sauerstoff erzeugt und dieser Sauerstoff wird in der (nachfolgend definierten) Vorrichtung (V2) gemäß Schritt g) gemeinsam mit Wasserstoff in elektrischen Strom und gegebenenfalls Wärme umgewandelt, wobei der Sauerstoff vor seiner Verwendung in Schritt g) gegebenenfalls zwischengespeichert wird. Vorrichtungen zum Zwischenspeichern von Sauerstoff sind dem Fachmann bekannt.

Weiterhin ist es bevorzugt, dass in Schritt e) zusätzlich Sauerstoff erzeugt wird und dieser Sauerstoff zur Optimierung der Haltung von Masttieren in einem landwirtschaftlichen Betrieb, zur Beschleunigung des Pflanzenwachstums in einem landwirtschaftlichen Betrieb oder zur Ozonisierung bei der Trinkwasserreinigung verwendet wird.

Weiterhin ist es bevorzugt, dass in Schritt e) zusätzlich Wärme frei wird, und die freigewordene Wärme gegebenenfalls in einem landwirtschaftlichen Betrieb verwendet wird oder nach (dem nachfolgend definierten) Schritt f) geleitet wird, vorzugsweise wird die in Schritt e) freigesetzte Wärme zwischengespeichert, insbesondere in einem Erdwärmespeicher, bevor sie nach Schritt f) geleitet wird. Vorrichtungen zur Zwischenspeicherung von Wärme als solche sind dem Fachmann bekannt.

Weiterhin ist es bevorzugt, dass im erfindungsgemäßen Verfahren nach Schritt c) ein Schritt f) durchgeführt, wobei gemäß dem optionalen Verfahrensschritt f) die Freisetzung von Wasserstoff aus dem mit Wasserstoff beladenen Träger (T2) erfolgt.

Prinzipiell ist es möglich, dass der optionale Verfahrensschritt f) ebenfalls in dem Fermenter (F1) durchgeführt wird. Vorzugsweise wird der Verfahrensschritt f) in einer separaten Vorrichtung (V4) durchgeführt, die insbesondere ein chemischer Reaktor ist. Die erfindungsgemäßen Verfahrensschritte c), d) und f) werden also vorzugsweise jeweils in separaten Vorrichtungen durchgeführt. Prinzipiell ist es möglich, dass die Vorrichtung (V4) zur Durchführung des Schritts f) in einer von der Art her gleichen Vorrichtung durchgeführt wird wie die Vorrichtung (V3) zur Durchführung des erfindungsgemäßen Verfahrensschritts d). Geeignete Vorrichtungen zur Durchführung des erfindungsgemäßen Verfahrensschritts f) sind beispielsweise in DE-B 10 2013 223 589 offenbart.

Das Freisetzen des Wasserstoffs gemäß Verfahrensschritt f) erfolgt vorzugsweise in einem druckstabilen chemischen Reaktor bei einer Prozesstemperatur zwischen 100°C und 450°C, bevorzugt zwischen 150°C und 420°C und insbesondere zwischen 180°C und 390°C. Der Prozessdruck liegt zwischen 0,1 und 30 bar, insbesondere zwischen 1 und 10 bar, wobei insbesondere ein metallhaltiger Katalysator eingesetzt werden kann, der insbesondere Platin und/oder Palladium enthält. Wesentlich ist, dass der Katalysator geeignet ist, Wasserstoff, der vom LOHC-Träger (T2) abgegeben wird, als Wasserstoffgas freizusetzen. Neben Platin und/oder Palladium sind dafür insbesondere Metalle, wie Chrom, Eisen, Kobalt, Nickel, Kupfer, Iridium oder Ruthenium, geeignet.

Weiterhin ist es bevorzugt, dass bei der Freisetzung von Wasserstoff gemäß Schritt f) zusätzlich der Träger (T1) wiedergewonnen wird.

Weiterhin ist es bevorzugt, dass bei der Freisetzung von Wasserstoff gemäß Schritt f) zusätzlich der Träger (T1) wiedergewonnen wird und der Träger (T1) erneut zur Durchführung von Schritt d) verwendet wird.

Weiterhin ist es bevorzugt, dass bei der Freisetzung von Wasserstoff gemäß Schritt f) zusätzlich der Träger (T1) wiedergewonnen wird und anschließend der Träger (T1) in einer separaten Vorrichtung (V5) oder im gegebenenfalls im Inneren mit einer Schutzauskleidung versehenen gereinigten Fermenter (F1) gelagert wird. Vorzugsweise ist die Vorrichtung (V5) ein Fermenter (F2), der gegebenenfalls umgerüstet ist. Die Umrüstung erfolgt vorzugsweise nach den vorstehend beschriebenen Verfahrensschritten a) sowie gegebenenfalls b).

Weiterhin ist es erfindungsgemäß bevorzugt, dass nach Schritt f) ein optionaler Verfahrensschritt g) durchgeführt wird, wobei in Schritt g) die Überführung des in Schritt f) erhaltenen Wasserstoffs in eine Vorrichtung (V2) erfolgt, wobei in der Vorrichtung (V2) Wasserstoff in elektrischen Strom und gegebenenfalls in Wärme umgewandelt wird.

Die Vorrichtung (V2) als solche ist dem Fachmann bekannt. Vorzugsweise ist die Vorrichtung (V2) Bestandteil eines Blockheizkraftwerkes, ein Gasmotor, eine Brennstoffzelle, ein Verbrennungsmotor, ein Verbrennungsmotor mit angeschlossenem Generator, eine Turbine oder eine Wasserstoffturbine mit angeschlossenem Generator, vorzugsweise ein Gasmotor.

Die vorgenannten Vorrichtungen (V2) sind häufig bereits in einer konventionellen Biogasanlage enthalten und können in aller Regel ohne weitere Umrüstungen im erfindungsgemäßen Verfahren eingesetzt werden. Dies trifft insbesondere für ein Blockheizkraftwerk und/oder einen Gasmotor zu, die häufig in Biogasanlagen verwendet werden. Weiterhin ist es bevorzugt, dass die gegebenenfalls in Schritt g) erzeugte Wärme nach Schritt f) zurückgeleitet wird. Die in Schritt g) freigesetzte Wärme kann gegebenenfalls mit der, wie vorstehend bereits erwähnt, in Schritt e) optional anfallenden Wärme vereinigt und gegebenenfalls gemeinsam zwischengelagert werden. Sinngemäßes trifft auch auf die Wärme zu, die zusätzlich im erfindungsgemäßen Verfahren in Schritt d) freiwerden kann.

Vorzugsweise wird in Schritt d) zusätzlich Wärme frei und diese freigewordene Wärme wird in einem landwirtschaftlichen Betrieb verwendet, vorzugsweise zur Beheizung von Gewächshäusern. Ebenso ist es bevorzugt, dass in Schritt d) zusätzlich Wärme frei wird und die freigewordene Wärme nach Schritt f) geleitet wird, wobei die Wärme vor ihrer Verwendung in Schritt f) vorzugsweise zwischengespeichert wird, insbesondere in einem Erdwärmespeicher zwischengespeichert wird.

In einer weiteren bevorzugten Ausführungsform der vorliegenden Erfindung wird das Verfahren in einer Anlage durchgeführt, die weiterhin zur Erzeugung von Biogas eingesetzt werden kann. Die Erzeugung von Biogas kann dabei parallel oder zeitversetzt zum erfindungsgemäßen Verfahren zum Speichern und Lagern von Wasserstoff bzw. bei der gegebenenfalls nachfolgend durchgeführten Freisetzung von Wasserstoff und der damit verbundenen Erzeugung von elektrischem Strom und gegebenenfalls von Wärme durchgeführt werden. Ebenso ist es denkbar, dass die Erzeugung von Biogas nur für ein bestimmtes Zeitintervall parallel zum Verfahren zum Speichern und Lagern von Wasserstoff durchgeführt wird.

Vorzugsweise werden die Erzeugung von Biogas einerseits und das Speichern und Lagern von Wasserstoff oder die Erzeugung von elektrischem Strom sowie gegebenenfalls von Wärme andererseits parallel auf derselben Anlage durchgeführt.

Weiterhin ist es bevorzugt, dass die Erzeugung von Biogas in einem separaten Fermenter (F3) durchgeführt wird, der nicht Bestandteil des gegebenenfalls mit einer Schutzauskleidung versehenen gereinigten Fermenters (F1) zur Lagerung des mit Wasserstoff beladenen Trägers (T2) ist.

Weiterhin ist es bevorzugt, dass der in Schritt e) erzeugte Wasserstoff teilweise und/oder für eine bestimmte Zeitspanne zur Erzeugung von Biogas, insbesondere zur Methanisierung, eingesetzt wird, wobei der Wasserstoff vorzugsweise in den separaten Fermenter (F3) eingeleitet wird und dort mit CO₂ abreagiert.

Weiterhin ist es bevorzugt, dass der in Schritt f) freigesetzte Wasserstoff teilweise und/oder für eine bestimmte Zeitspanne zur Erzeugung von Biogas, insbesondere zur Methanisierung, eingesetzt wird, wobei der Wasserstoff vorzugsweise in den separaten Fermenter (F3) eingeleitet wird und dort mit CO₂ abreagiert.

Die in den vorgenannten Optionen erwähnten Zeitspannen sind prinzipiell beliebig. Es kann sich dabei um eine oder mehrere Stunden, einen oder mehrere Tage oder sogar um noch längere Zeiträume, wie Wochen und Monate, handeln. Dies hängt ganz vom jeweiligen Bedarf der Erzeugung von Biogas, der Speicherung von Wasserstoff und/oder der Erzeugung von elektrischem Strom ab.

Ein weiterer Gegenstand der vorliegenden Erfindung ist ein Fermenter (F1) als solcher, der im Inneren mit mindestens einer Schutzauskleidung versehen ist, wobei die Schutzauskleidung aus mindestens zwei Schichten aufgebaut ist. Bei dem Fermenter (F1) als solchem handelt es sich somit um einen Fermenter, der vorzugsweise durch das vorstehend beschriebene Verfahren zur Umrüstung einer Biogasanlage unter Berücksichtigung der Verfahrensschritte a) und b) hergestellt worden ist. Die vorstehend im Zusammenhang mit den Verfahrensschritten a) und b) erwähnten Definitionen und Merkmale, inklusive der entsprechenden bevorzugten, mehr bevorzugten, noch mehr bevorzugten und besonders bevorzugten Definitionen gelten somit auch für den Fermenter (F1) als solchen.

Der Fermenter (F1) als solcher kann darüber hinaus noch weitere Einrichtungselemente/Vorrichtungen, insbesondere in seinem Inneren, enthalten, wie beispielsweise Rührwerke, Heizsysteme, Leitungen oder Zuführ- und/oder Entnahmeelemente, beispielsweise in Form von Einlässen. Vorzugsweise enthält der Fermenter (F1) als solcher, der im Inneren mit mindestens einer Schutzauskleidung versehen ist, kein Rührwerk und/oder die entsprechenden Bedienelemente. Solche Einrichtungselemente/Vorrichtungen als solche sind dem Fachmann bekannt. Dies gilt auch für die nachfolgend aufgeführten Einrichtungselemente/Vorrichtungen, wie beispielsweise eine Druckkontrolleinrichtung. Sofern solche zusätzlichen Einrichtungselemente/Vorrichtungen, wie eine Druckkontrolleinrichtung, vorhanden sind, können etwaige auftretende Leckagen besonders gut überwacht bzw. festgestellt werden.

Vorzugsweise sind die Wände und/oder der Boden im Inneren des Fermenters (F1) aus Beton, Stahl oder Edelstahl, vorzugsweise aus Beton, hergestellt.

Vorzugsweise ist das Dach im Inneren des Fermenters (F1) aus mindestens einem Kunststoff, vorzugsweise aus Polyvinylchlorid (PVC) oder Polyethylen (PE), hergestellt. Weiterhin ist es dabei bevorzugt, dass das PVC in Folienform und/oder der Kunststoff, insbesondere das PE, Garne oder Fasern enthält.

Weiterhin ist ein Fermenter (F1) bevorzugt, wobei
i) die Schutzauskleidung den Boden und/oder die Wände im Inneren des gereinigten Fermenters (F1) vollständig oder zumindest größtenteils bedeckt, und/oder
ii) die Schutzauskleidung aus mindestens einem Kunststoff hergestellt ist, vorzugsweise ist der Kunststoff ausgewählt aus einem Polyalkylen, einem Epoxidharz, einem Polyurethan und/oder einer Plastikfolie, mehr bevorzugt ist der Kunststoff ausgewählt aus einem Polyethylen (PE), einem HD (high density)-Polyethylen (HD-PE), einem Polypropylen (PP) und/oder einem Polyurethan (PU), und/oder
iii) die Schutzauskleidung mit Glasfasern verstärkt ist, vorzugsweise ist die Schutzauskleidung aus mindestens einem glasfaserverstärkten Kunststoff hergestellt, und/oder
iv) die Schutzauskleidung aus mindestens zwei Schichten aufgebaut ist und mit einer zusätzlichen Druckkontrolleinrichtung versehen ist, vorzugsweise befindet sich ein Gas, insbesondere Luft, zwischen zwei Schichten der Schutzauskleidung, vorzugsweise ist das Gas unter Druck, und/oder
v) eine mindestens zwei Schichten aufweisende Schutzauskleidung, die eine Schicht aus Polyethylen und eine Schicht aus HD-Polyethylen umfasst, so an die Wände und/oder den Boden im Inneren des Fermenters (F1) angebracht wird, dass die Schicht aus HD-Polyethylen sich auf der von den Wänden und/oder dem Boden abgewandten Seite der Schutzauskleidung im Inneren des Fermenters (F1) befindet, und/oder
vi) die Wände und/oder der Boden im Inneren des Fermenters (F1) aus Beton hergestellt sind und eine Schutzauskleidung, die aus einem Polypropylen oder einem Polyurethan hergestellt ist, so auf den Beton aufgebracht wird, dass die Schicht der Schutzauskleidung, die aus einem Polypropylen oder einem Polyurethan hergestellt ist, direkt an den Beton angebracht ist.

Ebenso ist es bevorzugt, dass in das Innere des Fermenters (F1) zwei räumlich getrennte Bereiche eingebaut werden, wobei vorzugsweise die räumliche Trennung durch Einbau einer schwimmenden Trennwand in horizontaler Richtung oder durch Einbau einer Trennwand in vertikaler Richtung im Inneren des Fermenters (F1) erfolgt, wobei die Trennwand gegebenenfalls mit mindestens einer Schutzauskleidung versehen ist, besonders bevorzugt erfolgt die räumliche Trennung durch Einbau einer Trennwand in vertikaler Richtung im Inneren des Fermenters (F1), wobei die Trennwand gegebenenfalls mit mindestens einer Schutzauskleidung versehen ist.

Ein weiterer Gegenstand der vorliegenden Erfindung ist somit auch die Verwendung des vorstehend beschriebenen Fermenters (F1) zum Speichern und/oder Lagern von Wasserstoff, als Bestandteil einer Biogasanlage oder als Bestandteil einer Anlage zur Erzeugung von elektrischem Strom und gegebenenfalls von Wärme.

Ein weiterer Gegenstand der vorliegenden Erfindung ist somit auch eine Biogasanlage oder Anlage zur Erzeugung von elektrischem Strom und gegebenenfalls von Wärme, enthaltend mindestens einen Fermenter (F1) gemäß den vorstehenden Ausführungen.

## Patentansprüche

1. Verfahren zur Umrüstung einer Biogasanlage in eine Anlage zum Lagern von Wasserstoff umfassend die nachfolgenden Schritte a), c) und gegebenenfalls b):
a) Reinigen des Inneren von mindestens einem Fermenter (F1) der Biogasanlage,
b) gegebenenfalls Anbringen von mindestens einer Schutzauskleidung im Inneren des gereinigten Fermenters (F1),
c) Einbringen und Lagern eines mit Wasserstoff beladenen Trägers (T2) in den gegebenenfalls im Inneren mit einer Schutzauskleidung versehenen gereinigten Fermenter (F1).

2. Verfahren gemäß Anspruch 1, **dadurch gekennzeichnet, dass**
i) Schritt b) durchgeführt wird, und/oder
ii) die Wände und/oder der Boden im Inneren des Fermenters (F1) aus Beton, Stahl oder Edelstahl, vorzugsweise aus Beton, hergestellt sind, und/oder
iii) das Dach im Inneren des Fermenters (F1) aus mindestens einem Kunststoff, vorzugsweise aus Polyvinylchlorid (PVC) oder Polyethylen (PE), hergestellt ist, und/oder
iv) ein im Inneren des Fermenters (F1) vorhandenes Rührwerkzeug vor Durchführung von Schritt c) oder gegebenenfalls vor Durchführung von Schritt b) ausgebaut wird, und/oder
v) die Wände und/oder der Boden im Inneren des Fermenters (F1) aus Beton hergestellt sind und Schritt b) durchgeführt wird, oder
vi) die Wände und/oder der Boden im Inneren des Fermenters (F1) aus Edelstahl hergestellt sind und Schritt b) nicht durchgeführt wird.

3. Verfahren gemäß Anspruch 1 oder 2, **dadurch gekennzeichnet, dass**
i) die Schutzauskleidung den Boden und/oder die Wände im Inneren des gereinigten Fermenters (F1) vollständig oder zumindest größtenteils bedeckt, und/oder
ii) die Schutzauskleidung aus mindestens einem Kunststoff hergestellt ist, vorzugsweise ist der Kunststoff ausgewählt aus einem Polyalkylen, einem Epoxidharz, einem Polyurethan und/oder einer Plastikfolie, mehr bevorzugt ist der Kunststoff ausgewählt aus einem Polyethylen (PE), einem HD (high density)-Polyethylen (HD-PE), einem Polypropylen (PP) und/oder einem Polyurethan (PU), und/oder
iii) die Schutzauskleidung mit Glasfasern verstärkt ist, vorzugsweise ist die Schutzauskleidung aus mindestens einem glasfaserverstärkten Kunststoff hergestellt, und/oder
iv) die Schutzauskleidung aus mindestens zwei Schichten aufgebaut ist und gegebenenfalls mit einer zusätzlichen Druckkontrolleinrichtung versehen ist, vorzugsweise befindet sich ein Gas, insbesondere Luft, zwischen zwei Schichten der Schutzauskleidung, vorzugsweise ist das Gas unter Druck, und/oder
v) eine mindestens zwei Schichten aufweisende Schutzauskleidung, die eine Schicht aus Polyethylen und eine Schicht aus HD-Polyethylen umfasst, so an die Wände und/oder den Boden im Inneren des Fermenters (F1) angebracht wird, dass die Schicht aus HD-Polyethylen sich auf der von den Wänden und/oder dem Boden abgewandten Seite der Schutzauskleidung im Inneren des Fermenters (F1) befindet, und/oder
vi) die Wände und/oder der Boden im Inneren des Fermenters (F1) aus Beton hergestellt sind und eine Schutzauskleidung, die aus einem Polypropylen oder einem Polyurethan hergestellt ist, so auf den Beton aufgebracht wird, dass die Schicht der Schutzauskleidung, die aus einem Polypropylen oder einem Polyurethan hergestellt ist, direkt an den Beton angebracht ist.

4. Verfahren gemäß einem der Ansprüche 1 bis 3, **dadurch gekennzeichnet, dass** in das Innere des gereinigten Fermenters (F1) zwei räumlich getrennte Bereiche eingebaut werden, wobei vorzugsweise die räumliche Trennung durch Einbau einer schwimmenden Trennwand in horizontaler Richtung oder durch Einbau einer Trennwand in vertikaler Richtung im Inneren des Fermenters (F1) erfolgt, wobei die Trennwand gegebenenfalls mit mindestens einer Schutzauskleidung versehen ist, besonders bevorzugt erfolgt die räumliche Trennung durch Einbau einer Trennwand in vertikaler Richtung im Inneren des Fermenters (F1), wobei die Trennwand gegebenenfalls mit mindestens einer Schutzauskleidung versehen ist.

5. Verfahren gemäß einem der Ansprüche 1 bis 4, **dadurch gekennzeichnet, dass**
i) Schritt a) einen Teilschritt a1) umfasst, wobei vom Inneren des Fermenters (F1) mindestens eine Oberflächenschicht zumindest teilweise entfernt wird, und/oder
ii) Schritt a) einen Teilschritt a2) umfasst, wobei im Inneren des Fermenters (F1) mindestens eine alkalische Wäsche durchgeführt wird, und/oder
iii) Schritt a) einen Teilschritt a3) umfasst, wobei aus dem Inneren des Fermenters (F1) sowie gegebenenfalls aus seinen Zuleitungen alle Substanzen, die sich infolge der Erzeugung von Biogas im Inneren des Fermenters (F1) oder gegebenenfalls in seinen Zuleitungen befinden, entfernt werden, und/oder
iv) Schritt a) einen Teilschritt a4) umfasst, wobei im Anschluss an mindestens einen der Teilschritte a1) bis a3) mit Wasser oder einem wässrigen Reinigungsmittel gespült sowie gegebenenfalls danach ein Trocknungsschritt durchgeführt wird.

6. Verfahren gemäß Anspruch 5, **dadurch gekennzeichnet, dass**
i) das Entfernen gemäß Teilschritt a3) durch Ablassen, vorzugsweise durch Absaugen, der Substanzen erfolgt, und/oder
ii) in Teilschritt a1) das Entfernen durch Abschleifen oder Abtragen, vorzugsweise durch Abschleifen, von mindestens einer Oberflächenschicht erfolgt, und/oder
iii) in Teilschritt a1) mindestens eine Oberflächenschicht vom Boden und/oder den Wänden im Inneren des Fermenters (F1) vollständig entfernt wird, und/oder
iv) zur Reinigung der Decke des Fermenters (F1) der Teilschritt a2) und/oder der Teilschritt a4) durchgeführt wird, und/oder
v) die Reinigung einer Zuleitung des Fermenters (F1) bis zur ersten Absperrvorrichtung der Zuleitung erfolgt und/oder eine Zuleitung des Fermenters (F1) im Inneren des Fermenters (F1) verschlossen wird, vorzugsweise durch Anbringen eines Stopfens, und/oder
vi) ein im Inneren des Fermenters (F1) vorhandenes Rührwerkzeug vor Durchführung von einem der Teilschritte a1), a2) oder a4) und/oder nach Durchführung des Teilschritts a3) ausgebaut wird.

7. Verfahren gemäß einem der Ansprüche 1 bis 6, **dadurch gekennzeichnet, dass** vor Schritt c) ein Schritt d) durchgeführt wird:
d) der mit Wasserstoff beladene Träger (T2) gemäß Schritt c) durch Inkontaktbringen eines Trägers (T1) mit Wasserstoff erhalten wird, um den Wasserstoff zu speichern, wobei vorzugsweise
i) in Schritt d) Wasserstoff chemisch oder physisorptiv, vorzugsweise chemisch, an den Träger (T1) gebunden wird, und/oder
ii) der Träger (T1) eine ungesättigte Verbindung, bevorzugt eine Verbindung mit mindestens einer Kohlenstoff-Kohlenstoff-Doppelbindung, mehr bevorzugt eine aromatische oder hetero-aromatische Verbindung, noch mehr bevorzugt ein N-Alkylcarbazol, Toluol, Dibenzyltoluol, Benzyltoluol, Naphthalin oder ein Azaborin, besonders bevorzugt Dibenzyltoluol, ist, und/oder
iii) in Schritt d) der mit Wasserstoff beladene Träger (T2) durch zumindest teilweise oder vollständige Hydrierung, vorzugsweise durch vollständige Hydrierung, mit Wasserstoff aus dem Träger (T1) erhalten wird.

8. Verfahren gemäß Anspruch 7, **dadurch gekennzeichnet, dass** vor Schritt d) ein Schritt e) durchgeführt wird:
e) Erzeugung von Wasserstoff in einer Vorrichtung (V6), vorzugsweise in einem Elektrolyseur, unter Verwendung von Wasser und elektrischem Strom.

9. Verfahren gemäß einem der Ansprüche 1 bis 8, **dadurch gekennzeichnet, dass** nach Schritt c) ein Schritt f) durchgeführt wird:
f) Freisetzung von Wasserstoff aus dem mit Wasserstoff beladenen Träger (T2).

10. Verfahren gemäß Anspruch 9, **dadurch gekennzeichnet, dass** nach Schritt f) ein Schritt g) durchgeführt wird:
g) Überführung des in Schritt f) erhaltenen Wasserstoffs in eine Vorrichtung (V2), wobei in der Vorrichtung (V2) Wasserstoff in elektrischen Strom und gegebenenfalls in Wärme umgewandelt wird, wobei vorzugsweise
i) die Vorrichtung (V2) Bestandteil eines Blockheizkraftwerkes, ein Gasmotor, eine Brennstoffzelle, ein Verbrennungsmotor, ein Verbrennungsmotor mit angeschlossenem Generator, eine Turbine oder eine Wasserstoffturbine mit angeschlossenem Generator, vorzugsweise ein Gasmotor, ist, und/oder
ii) die gegebenenfalls in Schritt g) erzeugte Wärme nach Schritt f) zurückgeleitet wird.

11. Verfahren gemäß einem der Ansprüche 9 oder 10, **dadurch gekennzeichnet, dass**
i) bei der Freisetzung von Wasserstoff gemäß Schritt f) zusätzlich der Träger (T1) wiedergewonnen wird, und/oder
ii) bei der Freisetzung von Wasserstoff gemäß Schritt f) zusätzlich der Träger (T1) wiedergewonnen wird und der Träger (T1) erneut zur Durchführung von Schritt d) verwendet wird, und/oder
iii) bei der Freisetzung von Wasserstoff gemäß Schritt f) zusätzlich der Träger (T1) wiedergewonnen wird und anschließend der Träger (T1) in einer separaten Vorrichtung (V5) oder im gegebenenfalls mit einer Schutzauskleidung versehenen gereinigten Fermenter (F1) gelagert wird, vorzugsweise ist die Vorrichtung (V5) ein Fermenter (F2), der gegebenenfalls umgerüstet ist, und/oder
iv) Schritt f) in einer separaten Vorrichtung (V4) durchgeführt wird, die vorzugsweise ein chemischer Reaktor ist.

12. Verfahren gemäß einem der Ansprüche 1 bis 11, **dadurch gekennzeichnet, dass** das Verfahren in einer Anlage durchgeführt wird, die weiterhin zur Erzeugung von Biogas eingesetzt werden kann, wobei vorzugsweise
i) die Erzeugung von Biogas einerseits und das Speichern und Lagern von Wasserstoff oder die Erzeugung von elektrischem Strom sowie gegebenenfalls von Wärme andererseits parallel auf derselben Anlage durchgeführt werden, und/oder
ii) die Erzeugung von Biogas in einem separaten Fermenter (F3) durchgeführt wird, der nicht Bestandteil des gegebenenfalls mit einer Schutzauskleidung versehenen gereinigten Fermenters (F1) zur Lagerung des mit Wasserstoff beladenen Trägers (T2) ist.

13. Fermenter (F1), der im Inneren mit mindestens einer Schutzauskleidung versehen ist, wobei die Schutzauskleidung aus mindestens zwei Schichten aufgebaut ist, wobei vorzugsweise
i) die Schutzauskleidung den Boden und/oder die Wände im Inneren des gereinigten Fermenters (F1) vollständig oder zumindest größtenteils bedeckt, und/oder
ii) die Schutzauskleidung aus mindestens einem Kunststoff hergestellt ist, vorzugsweise ist der Kunststoff ausgewählt aus einem Polyalkylen, einem Epoxidharz, einem Polyurethan und/oder einer Plastikfolie, mehr bevorzugt ist der Kunststoff ausgewählt aus einem Polyethylen (PE), einem HD (high density)-Polyethylen (HD-PE), einem Polypropylen (PP) und/oder einem Polyurethan (PU), und/oder
iii) die Schutzauskleidung mit Glasfasern verstärkt ist, vorzugsweise ist die Schutzauskleidung aus mindestens einem glasfaserverstärkten Kunststoff hergestellt, und/oder
iv) die Schutzauskleidung aus mindestens zwei Schichten aufgebaut ist und mit einer zusätzlichen Druckkontrolleinrichtung versehen ist, vorzugsweise befindet sich ein Gas, insbesondere Luft, zwischen zwei Schichten der Schutzauskleidung, vorzugsweise ist das Gas unter Druck, und/oder
v) eine mindestens zwei Schichten aufweisende Schutzauskleidung, die eine Schicht aus Polyethylen und eine Schicht aus HD-Polyethylen umfasst, so an die Wände und/oder den Boden im Inneren des Fermenters (F1) angebracht wird, dass die Schicht aus HD-Polyethylen sich auf der von den Wänden und/oder dem Boden abgewandten Seite der Schutzauskleidung im Inneren des Fermenters (F1) befindet, und/oder
vi) die Wände und/oder der Boden im Inneren des Fermenters (F1) aus Beton hergestellt sind und eine Schutzauskleidung, die aus einem Polypropylen oder einem Polyurethan hergestellt ist, so auf den Beton aufgebracht wird, dass die Schicht der Schutzauskleidung, die aus einem Polypropylen oder einem Polyurethan hergestellt ist, direkt an den Beton angebracht ist.

14. Verwendung eines Fermenters (F1) gemäß Anspruch 13 zum Speichern und/oder Lagern von Wasserstoff, als Bestandteil einer Biogasanlage oder als Bestandteil einer Anlage zur Erzeugung von elektrischem Strom und gegebenenfalls von Wärme.

15. Biogasanlage oder Anlage zur Erzeugung von elektrischem Strom und gegebenenfalls von Wärme enthaltend mindestens einen Fermenter (F1) gemäß Anspruch 13.
